# NEUE EUROPÄISCHE PATENTSCHRIFT

(11) **EP 0 356 725 B2**
(45) Veröffentlichungstag und Bekanntmachung des Hinweises auf die Entscheidung über den Einspruch: **03.02.1999**
(45) Hinweis auf die Patenterteilung: 21.12.1994
(21) Anmeldenummer: 89114029.5
(22) Anmeldetag: 29.07.1989
(51) Int. Cl.: C08G 65/32, C08G 65/28, C10L 1/22, C07C 233/18

(54) **Polyetheramine oder Polyetheraminderivate enthaltende Kraftstoffe für Ottomotoren**
Fuels for spark ignition engines containing polyether amines or polyether amine derivatives
Carburants pour moteurs à allumage par étincelle contenant des polyétheramines ou des dérivés de polyétheramines

(30) Priorität: 05.08.1988 DE 3826608
(43) Veröffentlichungstag der Anmeldung: 07.03.1990
(73) Patentinhaber: BASF Aktiengesellschaft, 67063 Ludwigshafen (DE)
(72) Erfinder: Rath, Hans Peter, Dr., D-6718 Gruenstadt (DE); Mach, Helmut, Dr., D-6900 Heidelberg (DE); Oppenlaender, Knut, Dr., D-6700 Ludwigshafen (DE); Schoenleben, Willibald, Dr., D-6900 Heidelberg (DE); Vogel, Hans-Henning, Dr., D-6710 Frankenthal (DE)

(56) Entgegenhaltungen:
- EP-A- 0 047 206
- EP-A- 0 100 665
- EP-A- 0 289 785
- WO-A-85/00827
- CH-A- 191 406
- FR-A- 2 334 655
- FR-A- 2 334 656
- US-A- 2 229 024
- US-A- 3 440 029
- US-A- 4 144 035
- US-A- 4 247 301
- US-A- 4 304 690
- US-A- 4 609 377

## Beschreibung

Die Erfindung betrifft Kraftstoffe für Ottomotoren, die geringe Mengen eines Polyetheramins und/oder Polyetheraminderivats enthalten, wobei das Polyetheramin durch reduktive Aminierung von Alkylphenolgestarteten Polyethern und die Polyetheraminderivate durch Umsetzung der Polyetheramine mit Alkylenoxiden oder Carbonsäuren hergestellt sind.

Polyetheramine sind als Kraftstoffadditive zur Reinhaltung und Reinigung von Vergasern, Einspritzdüsen und Ventilen bekannt und z.B. Gegenstand der PCT-Anmeldung WO 85/01956 oder der EP-B1 0 100 665.

Dort sind Verbindungen beschrieben, die ausgehend von Ethylenchlorhydrin, Alkoxylierung, Verätherung der Endhydroxylgruppe und Ersatz des Chloratoms durch eine Aminogruppe hergestellt werden.

Obgleich diese Polyetheramine ausgezeichnete Ventilreiniger mit ausgeprägtem Reinigungseffekt im Einlaßsystem des Motors sind, haben sie den Nachteil eines von der Herstellung verbleibenden Chlorgehalts. Kraftstoff- oder Ölzusätze, die Chlor enthalten, sind jedoch aus Gründen der Korrosion und des Umweltschutzes unerwünscht.

Es bestand daher die Aufgabe chlorfreie, als Kraftstoffadditive geeignete Polyetheramine bereitzustellen. Es bestand weiterhin die Aufgabe, die bekannten Polyetheramine in ihrer Wirkung zu verbessern bzw. mit geringerer Dosis die gleiche Wirkung zu erzielen.

Es wurde nun überraschenderweise gefunden, daß Kraftstoffe für Ottomotoren, die geringe Mengen Polyetheramine und/oder Polyetheraminderivate enthalten, eine sehr gute Ventil- und Vergaserreinigungswirkung haben und kein Chlor aufweisen, wenn die Polyetheramine und Polyetheraminderivate solche der allgemeinen Formel 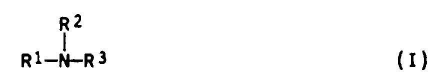 oder 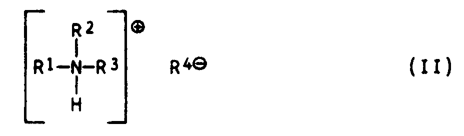 sind, worin R¹ einen Alkylphenolpolyetherrest der allgemeinen Formel 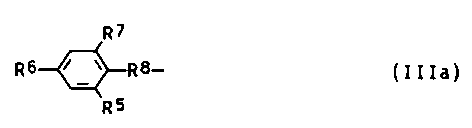 oder einen Alkylcyclohexylpolyetherrest der allgemeinen Formel 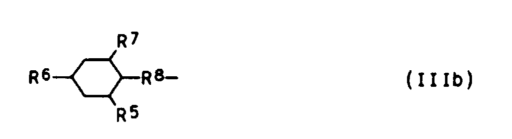 bedeutet, R² und R³ gleich oder verschieden sein können und für Wasserstoff, den Alkylphenolpolyetherrest (IIIa), den Alkylcyclohexylpolyetherrest (IIIb), den Acylrest einer Carbonsäure mit 2 bis 24 Kohlenstoffatomen oder den Hydroxyalkylrest der allgemeinen Formel 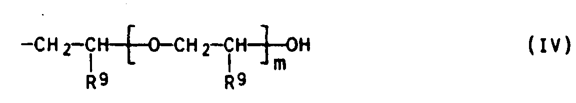 stehen oder R² für einen Alkylrest mit 1 bis 20 Kohlenstoffatomen steht und R³ für Wasserstoff, den Alkylphenolpolyetherrest (IIIa), den Alkylcyclohexylpolyetherrest (IIIb), den Acylrest einer Carbonsäure mit 2 bis 24 Kohlenstoffatomen oder den Hydroxyalkylrest (IV) steht,
R⁴ den Carboxylatrest einer Carbonsäure mit 2 bis 24 Kohlenstoffatomen bedeutet, R⁵, R⁶ und R⁷ gleich oder verschieden sein können und für Wasserstoff oder einen Kohlenwasserstoffrest mit 1 bis 30 Kohlenstoffatomen stehen, R⁸ für eine Polyetherkette aus Alkenoxiden mit 3 bis 8 Kohlenstoffatomen oder Gemischen dieser Alkenoxide mit 2 bis 100 Alkenoxideinheiten in der Kette steht und R⁹ einen Kohlenwasserstoffrest mit 1 bis 6 Kohlenstoffatomen und m die Zahlen 0 bis 5 bedeuten, wobei das mittlere Molekulargewicht Mₙ der Polyetheramine oder Polyetheraminderivate (I) bzw. (II) 500 bis 8000 beträgt und die Polyetheramine oder die den Polyetheraminderivaten zugrunde liegenden Polyetheramine durch reduktive Aminierung von Alkylphenolpolyethern der allgemeinen Formel 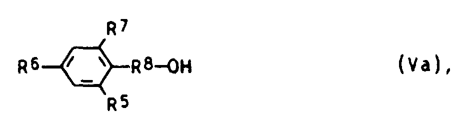 oder von Alkylcyclohexanolen der allgemeinen Formel 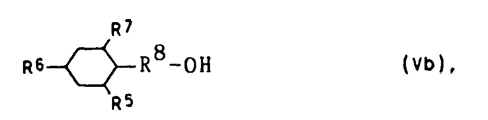 worin R⁵, R⁶, R⁷ und R⁸ die genannten Bedeutungen haben, mit Ammoniak oder primären Aminen hergestellt sind.

Die erfindungsgemäß Zu verwendenden Polyetheramine und Polyetheraminderivate werden im allgemeinen in mehreren Stufen synthetisiert. In einem ersten Schritt stellt man zweckmäßig durch Alkoxylierung mit Alkenoxiden mit 3 bis 8 Kohlenstoffatomen oder Gemischen dieser Alkenoxide von Alkylphenolen der allgemeinen Formel 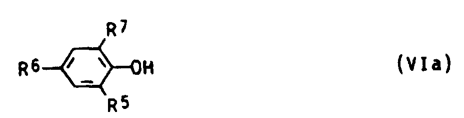 oder von Alkylcyclohexanolen der allgemeinen Formel 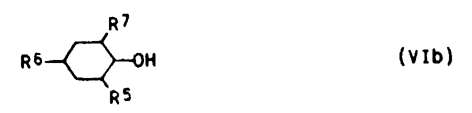 worin R⁵, R⁶ und R⁷ die vorstehend genannten Bedeutungen haben, in an sich bekannter Weise Alkylphenolpolyether der allgemeinen Formel (Va) bzw. Alkylcyclohexylpolyether der allgemeinen Formel (Vb) her. Die Alkoxilierung wird, gegebenenfalls in Gegenwart von Alkali wie Kalilauge, Natronlauge, Natriummethylat, zweckmäßig bei erhöhten Temperaturn, beispielsweise bei 80 bis 140°C, vorzugsweise 100 bis 120°C durchgeführt.

Als Reste R⁵, R⁶ und R⁷ kommen dabei Wasserstoff und lineare, vorzugsweise jedoch verzweigte Kohlenwasserstoffreste mit 1 bis 30 Kohlenstoffatomen in Betracht, wobei von den Kohlenwasserstoffresten Methylreste und verzweigte Kohlenwasserstoffreste mit 3 bis 16 Kohlenstoffatomen besonders bevorzugt werden. Im einzelnen kommen als Starter neben den Kresolen alkylierte Phenole und Kresole in Betracht.

Beispielsweise seien genannt lsobutylphenol, -kresol, Diisobutylphenol, -kresol, tert-Butylphenol, -kresol, Di-tert.-butylphenol, -kresol, lsooctylphenol, Diisooctylphenol, lsononylphenol, Diisononylphenol, Isododecylphenol, Diisododecylphenol oder Mischungen daraus.

Die als Starter zu verwendenden Alkylphenole erhält man in an sich bekannter Weise, z.B. durch Alkylierung von Phenolen, Kresolen oder Dimethylphenolen mit den entsprechenden Olefinen. Die ebenfalls als Starter zu verwendenden Alkylcyclohexanole erhält man beispielsweise durch Kernhydrierung von entsprechenden Alkylphenolen.

Die Alkoxylierung von den Alkylphenolen bzw. von den Alkylcyclohexanolen mit den Alkenoxiden mit 3 bis 8, vorzugsweise 3 bis 6 Kohlenstoffatomen, insbesondere mit Propylenoxid und/oder Butylenoxid wie 1,2-Butylenoxid, 2,3-Butylenoxid, lsobutylenoxid, wobei die Umsetzung mit den Butylenoxiden bevorzugt ist, erfolgt in an sich bekannter Weise. Eine allgemeine Herstellvorschrift wird weiter unten angegeben. Beispielsweise erfolgt die Umsetzung nur mit einem Alkylenoxid, z.B. mit Propylenoxid oder mit Butylenoxid, oder mit Gemischen von Alkylenoxiden, z.B. einem Gemisch von Propylenoxid und Butylenoxid. Die Umsetzungen mit dem Alkylenoxid, z.B. Propylenoxid oder Butylenoxid, oder einem Gemisch von Alkylenoxiden, z.B. einem Gemisch von Propylenoxid und Butylenoxid, kann in einer Stufe durchgeführt werden. Es kann jedoch auch vorteilhaft sein, die in der ersten Stufe erhaltenen Verbindungen in einer zweiten Stufe oder mehr als zwei Stufen, wobei zwei Stufen bevorzugt werden, mit weiterem Alkylenoxid wie Propylenoxid oder Butylenoxid oder einem Gemisch von Alkylenoxiden, z.B. einem Gemisch von Propylenoxid und Butylenoxid, umzusetzen. Die Menge an Alkylenoxid, z.B. Propylenoxid bzw. Butylenoxid, kann in weiteren Bereichen schwanken. In der Regel verwendet man 3 bis 100, vorzugsweise 5 bis 30 Mol Alkylenoxid pro Mol Starter. Die angewandte Menge und die Wahl des Alkylenoxids, im allgemeinen Propylenoxid oder Butylenoxid, hängt jedoch davon ab, welches Startermolekül verwendet wurde. Enthält das Startermolekül einen langkettigen hydrophoben Rest wie Diisododecylphenol, können größere Mengen an niedermolekularen Alkylenoxiden, vorzugsweise Propylenoxid, zur Anwendung kommen. Enthält das Startermolekül dagegen kürzerkettige hydrophobe Reste, kann es vorteilhaft sein, höhermolekulare Alkylenoxide, vorzugsweise Butylenoxid, zu verwenden bzw., z.B. in Mischungen von Propylenoxid und/oder Butylenoxid, den Anteil von Butylenoxid zu erhöhen.

Insgesamt gilt, daß die Alkenoxide und deren Menge so gewählt werden, daß eine Mindestlöslichkeit von 50 Gew.% in einem Kohlenwasserstoff, z.B. Toluol oder Mineralöl SN 100, zur Herstellung eines Master-batch gewährleistet ist.

In einer zweiten Stufe werden dann die Polyether im allgemeinen ohne weitere Vorbehandlung einer Aminierung nach an sich bekannten Methoden unterworfen. Unter Aminierung wird dabei die Umsetzung der Polyether (Va) und (Vb) mit Ammoniak oder primären Aminen verstanden, wobei im Polyether die endständige OH-Gruppe unter Wasserabspaltung durch eine Aminogruppe ersetzt wird. Die Methodik ist im einzelnen in Houben-Weyl 11/1, Kapitel 11b, Seiten 108- 134 beschrieben, worauf hiermit Bezug genommen wird.

Wie bei allen reduktiven Aminierungen können die noch freien Wasserstoffatome am Stickstoff der Aminogruppe durch weitere Polyetherreste (III) ersetzt werden, so daß in der Regel ein Gemisch von Aminen, z.B. bei der Aminierung mit Ammoniak ein Gemisch von primären, sekundären und tertiären Aminen, z.B. im Gewichtsverhältnis 6:3:1 entsteht. Bei der Aminierung mit primären Aminen bildet sich entsprechend ein Gemisch von sekundären und tertiären Aminen.

Die Aminierungsreaktion wird zweckmäßig bei Temperaturen zwischen 160 und 250°C und Drücken bis zu 600 bar, vorzugsweise 80 - 300 bar durchgeführt. Als Katalysatoren kommen vorzugsweise kobalt- und nickelhaltige Katalysatoren auf Trägern wie SiO₂ oder Al₂O₃, aber auch Raney Nickel oder Raney Kobalt selbst in Frage. Ein quantitativer Umsatz der OH-Gruppen ist für den Anwendungszweck nicht erforderlich, besonders dann, wenn die als Ausgangsverbindungen der Formel (Va) und (Vb) benutzten Polyether auch als Trägeröl für die Benzinadditivformulierung verwendet werden. Der Teilumsatz kann sogar vorteilhaft sein, da man höhere Raum-Zeit-Ausbeuten erhält. Im allgemeinen wendet man bei der Aminierung das Ammoniak bzw. das Amin im überschuß, z.B. im 10- bis 60fachen, vorzugsweise 15- bis 40fachen molaren überschuß, an. Dabei ist die Verwendung von Ammoniak bevorzugt. Als primäre Amine für die Aminierung werden solche mit einem Alkylrest mit 1 bis 20, vorzugsweise 1 bis 13, insbesondere 1 bis 8 Kohlenstoffatomen verwendet. Beispielsweise seien genannt Methyl-, Ethyl-, Butylamin.

Die durch Aminierung erhaltenen Polyetheramine können als solche den Kraftstoffen zugesetzt werden.

Sie können aber auch durch Umsetzung mit Alkylenoxiden oder Carbonsäuren in an sich bekannter Weise in die entsprechenden Derivate übergeführt werden, um in Form der Derivate den Kraftstoffen zugesetzt zu werden.

Bei der Derivatisierung der Polyetheramine mit Alkylenoxiden werden solche mit 3 bis 8, vorzugsweise 3 bis 4 Kohlenstoffatomen verwendet, beispielsweise Propylenoxid, Butylenoxid Die Alkoxilierung mit den Alkenoxiden erfolgt in an sich bekannter Weise, zweckmäßig in Gegenwart von Alkali wie Kalilauge, Natronlauge, Natriummethylat bei erhöhten Temperaturen, z.B. bei 120 bis 150°C. Eine allgemeine Herstellvorschrift wird weiter unten angegeben.

In den dabei erhaltenen Derivaten mit den Hydroxyalkylresten gemäß der allgemeinen Formel (IV) entspricht m den Zahlen 0 bis 5, wobei Derivate mit einem Kettenglied (m = 0) bevorzugt werden.

Die Derivatisierung der Polyetheramine durch Umsetzung mit Carbonsäuren kann einerseits als Neutralisation unter Bildung der entsprechenden Ammoniumsalze durchgeführt werden, wobei die Polyetheraminderivate gemäß der Formel (II) erhalten werden. Die Neutralisation erfolgt in an sich bekannter Weise. Eine allgemeine Herstellvorschrift wird weiter unten angegeben.

Die Derivatisierung der Polyetheramine mit Carbonsäuren kann weiter durch Amidierung unter Bildung der Carbonsäureamide erfolgen, d.h. zu Polyetheraminderivaten der Formel (I), in der R² und/oder R³ Acylreste darstellen. Die Amidierung erfolgt in an sich bekannter Weise. Eine allgemeine Herstellvorschrift wird weiter unten angegeben.

Als Carbonsäuren für die Neutralisation oder Amidierung werden im allgemeinen Monocarbonsäuren mit 2 bis 24, vorzugsweise 2 bis 10 Kohlenstoffatomen verwendet. Geeignete Carbonsäuren sind beispielsweise Essig-, Propion-, Valerian-, Capron-, Capryl-, Pelargon-, Caprin-, Laurin-, Palmitin-, Stearin-, Ölsäure, Isononansäure und 2-Ethylhexansäure.

Als Kraftstoff kommen verbleites und unverbleites Normal- und Superbenzin in Betracht. Die Benzine können auch andere Komponenten als Kohlenwasserstoffe, z.B. Alkohole wie Methanol, Ethanol, tert-Butanol sowie Ether, z.B. Methyltert-butylether enthalten. Neben den erfindungsgemäß zu verwendenden Polyetheraminen oder Polyetheraminderivaten enthalten die Kraftstoffe in der Regel noch weiter Zusätze wie Korrosionsinhibitoren, Stabilisatoren, Antioxydantien, Detergentien etc.

Korrosionsinhibitoren sind meist Ammoniumsalze organischer Carbonsäuren, die durch entsprechende Struktur der Ausgangsverbindungen zur Filmbildung neigen. Auch Amine zur Anhebung des pH-Wertes finden sich häufig in Korrosionsinhibitoren. Als Buntmetallkorrosionsschutz werden meist heterocyclische Aromaten eingesetzt.

Als Antioxidantien oder Stabilisatoren sind insbesondere Amine wie para-Phenylendiamin, Dicyclohexylamin, Morpholin oder Derivate dieser Amine zu nennen. Auch phenolische Antioxidantien wie 2,4-Di-tert-butylphenol oder 3,5-Di-tert-butyl-4-hydroxyphenylpropionsäure und deren Derivate werden Kraft- und Schmierstoffen zugesetzt.

Als Vergaser-, lnjector- und Ventildetergentien sind ferner gegebenenfalls Amide und Imide des Polyisobutylenbernsteinsäureanhydrids, Polybutenamine, Polybutenpolyamine sowie langkettige Carbonamide und -imide in den Kraftstoffen enthalten.

Als Trägeröle für Konzentrate der erfindungsgemäß zu verwendenden Polyetheramine oder deren Derivate können Mineralöle des Viskositätsbereiches SN 500-900, aber auch Brightstock und Syntheseöle wie Polyalphaolefin, Trimellithsäureester oder Polyether, insbesondere solche auf Alkylphenol- und Buten-oxidbasis, eingesetzt werden. Die Ester sollten möglichst langkettige, verzweigte Alkohole größer C₈, die Polyether vorzugsweise langkettige Starter und hohe PO- oder BuO-Gehalte, bezogen auf die Alkylenoxidmenge, im Molekül enthalten.

Die Kraftstoffe enthalten die Polyetheramine bzw. Polyetheraminderivate der Formel (I) oder (II) in der Regel in Mengen von 10 bis 2000 Gew.ppm bezogen auf das reine Polyetheramin oder Polyetheraminderivat. Meist sind aber bereits 20 bis 1000 Gew.ppm, vorzugsweise 40 bis 400 Gew.ppm, ausreichend.

Im folgenden wird die Herstellung der Polyetheramine und Polyetheraminderivate und ihre Wirkung im Motor im einzelnen erläutert.

### Herstellungsbeispiel

### 1. Umsetzung von Alkylphenol mit Alkylenoxiden

Die Herstellung der Polyether erfolgt nach bekannten Verfahren der Oxalkylierung mit Alkali.

In einem Autoklaven mit Rührer werden bezogen auf den gesamten Ansatz 0,1 Gew.% KOH fein pulverisiert im Alkylphenol unter Rühren verteilt und bei 200 mbar auf 130°C erhitzt. Dabei werden restliche Wasserspuren abgezogen. Dann wird der Autoklav geschlossen und Alkylenoxid so zudosiert, daß ein Druck von 6 bar nicht überschritten wird. Die Dosierung verschiedener Alkylenoxide kann gleichzeitig oder nacheinander erfolgen, so daß statistische Polyether oder Blöcke entstehen mit mehr oder minder scharfen Übergängen. Nach Zulaufende der Alkylenoxide fällt der Druck im Verlauf von 3 bis 10 h auf 2 bis 3 bar. Ist dieser Druck erreicht, kühlt man auf 80°C und entspannt über ein Membranventil und evakuiert bis auf 20 bis 30 mbar. Nach Aufrechterhalten des verminderten Drucks für ca. 1 h setzt man dann äquivalente Mengen sauren lonenaustauschers zur Entfernung von Kalium zu und filtriert.

### 2. Reduktive Aminierung der Polyether

Die gemäß Herstellungsbeispiel 1 hergestellten Polyether werden im allgemeinen ohne weitere Vorbehandlung der nachfolgenden Aminierung mit Ammoniak oder einem primären Amin unterworfen. Bei höheren Polyetherviskositäten empfiehlt sich jedoch eine Verdünnung mit Lösemittel, vorzugsweise verzweigte Aliphaten wie Isododekan, so daß man eine Viskosität von 50 - 200 mm²/s bei 20°C erhält. Beispielsweise werden die Polyether als solche oder in Lösung mit Ammoniak, der im allgemeinen im überschuß, z.B. im 2- bis 100-fachen, vorzugsweise 10- bis 80-fachen molaren überschuß verwendet wird, in Gegenwart von einem Hydrierkatalysator, z.B. Raney-Nickel, bei erhöhtem Druck, z.B. bei Drucken von 10 bis 300 bar, vorzugsweise 50 bis 250 bar, in einem Druckreaktor, z.B. einem Rollautoklaven, bei erhöhten Temperaturen, z.B. 80 bis 300°C, vorzugsweise 120 bis 250°C, mit Wasserstoff behandelt, z.B. über einen Zeitraum von 10 Minuten bis 10 Stunden. Nach dem Abkühlen trennt man den Katalysator durch Filtrieren ab, verdampft überschüssiges Ammoniak und destilliert das Reaktionswasser azeotrop oder unter leichtem Stickstoffstrom ab.

### 3. Derivatisierung von Polyetheraminen

Die gemäß Beispielen 1 und 2 hergestellten Polyetheramine lassen sich nach bekannten Verfahren mit Alkenoxiden oder Carbonsäuren derivatisieren. Dazu wird zweckmäßig zunächst die Aminzahl des Polyetheramins, z.B. mit 0,1 m HCl gegen Bromphenolblau bestimmt.

### a) Alkoxilierung

Die Umsetzung mit Epoxiden erfolgt im allgemeinen wie unter 1 beschrieben in Gegenwart alkalischer Katalysatoren wie KOH, NaOH, NaOCH₃ usw., die in Mengen von 0,1 bis 3 Gew.% bezogen auf Polyetheramin eingesetzt werden. Die Reaktionstemperaturen liegen je nach Epoxid bei 120 bis 150°C, die Reaktionszeiten bei 3 bis 6 Stunden, die Drucke bei 3 bis 6 bar. Die Umsetzung erfolgt in Druckrührbehältern unter portionsweiser Zugabe der Epoxide. Soll mehr als 1 bis 2 Mol Alkenoxid addiert werden, wird zweckmäßig mehrstufig, vorzugsweise zweistufig wie nachfolgend beschrieben, gearbeitet. Das primäre oder sekundäre Etheramin wird zunächst in Gegenwart geringer Mengen Wasser (3 bis 5 Gew.% bez. auf Etheramin) mit 1 bis 2 Mol Epoxid bei 100 bis 120°C umgesetzt. Man erhält im Falle sekundärer Amine vorwiegend die entsprechende N-Hydroxialkylverbindung, im Falle primärer Amine das entsprechende Bis-OH-alkylaminoderivat. Dann wird im Vakuum (z.B. 15 bis 30 mbar) bei z.B. 80 bis 100°C entwässert, mit geringen Mengen alkalischer Katalysatoren versetzt und zur Umsetzung mit der restlichen Epoxidmenge wie unter 1 bzw. 3a beschrieben verfahren. Die Produkte werden durch Amin- und Hydroxylzahl sowie durch die Gewichtszunahme charakterisiert.

### b) Neutralisation

Die Reaktionskomponenten werden zweckmäßig in stöchiometrischen Mengen unter Rühren und leichtem Erwärmen umgesetzt. Unter Umständen ist jedoch vorteilhaft, das Etheramin vorzulegen und die Carbonsäure portionsweise zuzugeben.

### c) Amidierung

Die Reaktion wird bei erhöhten Temperaturen, zweckmäßig bei 100 bis 180°C, z.B. bei 150 bis 160°C, unter Rühren der in stöchiometrischen Mengen eingesetzten Reaktionskomponenten mit oder ohne Lösemittel in Inertgasatmosphäre (N2) durchgeführt, wobei das Amin zweckmäßig vorgelegt und die Säurekomponente portionsweise zugegeben wird. Das Reaktionswasser wird bei lösungsmittelfreier Reaktionsführung während der Reaktion im Vakuum, z.B. bei 10 bis 100 mbar, oder durch azeotrope Destillation mit geeigneten Schleppmitteln, z.B. aromatische oder aliphatische Kohlenwasserstoffe, wie Toluol, Xylol, Schwerbenzin etc. entfernt, um eine möglichst vollständige Umsetzung zu erzielen.

Die Reaktionszeit beträgt im allgemeinen 4 bis 6 Stunden. Das Reaktionsende wird durch Bestimmung der Säurezahl (kleiner 3), der Aminzahl (kleiner 4) und der Menge des Reaktionswassers ermittelt.

### Beispiele

Nach den vorstehend unter 1, 2 und 3 angegebenen Methoden wurden folgende Produkte hergestellt:
A: lsononylphenol wird mit 1-Butenoxid im molaren Verhältnis 1:19 gemäß Herstellungsbeispiel 1 umgesetzt. Der erhaltene Polyether hat eine Viskosität von 220 mm²/s bei 40°C und ein rechnerisches Molekulargewicht von 1588. Dann wird entsprechend Herstellungsbeispiel 2 bei 100°C und 200 bar mit einem 50 molaren Ammoniak-überschuß an einem mit Nickel beschichteten Katalysator in Gegenwart von H₂ aminiert und bei einer Verweilzeit von 20 min ein Polyetheramin in einer Ausbeute von 94 % erhalten. Die Viskosität des Produktes ist 190 mm²/s bei 40°C, das Molekulargewicht 1587 und die Aminzahl 30,4 (theoretisch 37,6).
B: lsononylphenol wird mit 1-Butenoxid im molaren Verhältnis 1:8 gemäß Herstellungsbeispiel 1 umgesetzt. Der erhaltene Polyether hat eine Viskosität von 130 mm²/s bei 40°C und ein rechnerisches Molekulargewicht von 796. Dieser wird wie unter A beschrieben reduktiv aminiert. Man erhält Polyetheramin in einer Ausbeute von 96 % mit einer Viskosität von 100 mm²/s bei 40°C und einer Aminzahl von 68.
C: 2,6-Di-tert-butyl-p-kresol wird mit Propenoxid im molaren Verhältnis 1:9 gemäß Herstellungsbeispiel 1 umgesetzt. Der erhaltene Polyether hat eine Viskosität von 80 mm²/s bei 40°C und ein Molekulargewicht von 742. Dieser wird wie unter A beschrieben reduktiv aminiert, wobei die Ausbeute 93 % beträgt. Die Viskosität des Produktes ist 60 mm²/s bei 40°C, das Molekulargewicht 741 und die Aminzahl 70.
D: Dinonylphenol wird mit Propenoxid im molaren Verhältnis 1:8 gemäß Herstellungsbeispiel 1 umgesetzt. Der erhaltene Polyether hat eine Viskosität von 95 mm²/s bei 40°C und ein Molekulargewicht von 810. Der so erhaltene Polyether wird mit Ammoniak gemäß Herstellungsbeispiel 2 wie unter A beschrieben reduktiv aminiert. Man erhält Polyetheramin in einer Ausbeute von 96 % mit einer Viskosität von 80 mm²/s bei 40°C und einer Aminzahl von 66.
G: 100 Teile der gemäß Beispiel A hergestellten Verbindung werden mit 10 Teilen Isononansäure versetzt, wobei gemäß Methode 3b) ein Ammoniumsalz der überwiegend vorhandenen primären Amingruppen entsteht, d.h. das molare Verhältnis von Amin zu Isononansäure ist dabei 1:1.
H: 100 Teile der gemäß Beispiel A hergestellten Verbindung werden mit 10 Teilen Isononansäure versetzt und gemäß Methode 3c) in das Amid überführt. Das molare Verhältnis von Amin zu Isononansäure ist dabei 1:1.

### Durchführung der motorischen Prüfungen

Die motorischen Prüfungen mit den Additiven bzw. Additivpaketen wurden auf einem Daimler Benz M 102 E Motor mit nachstehend angegebenem Wechsellastprogramm durchgeführt.

| Wechsellastprogramm | | |
|---|---|---|
| Laufzeit (s) | Drehzahl (1/min) | Last (Nm) |
| 30 | 800 | 0 |
| 60 | 3000 | 8,34 |
| 60 | 1300 | 4,6 |
| 120 | 1 850 | 5,44 |

Die Laufzeit betrug 60 Stunden, die Zahl der Zyklen 800. Als Kraftstoff wurde unverbleites, alkoholhaltiges Superbenzin (3 % Methanol, 2 % tert. Butanol), als Motorenöl das Referenzöl des Opel Kadett Tests CEC-F-O2-T-79, RL 51 verwendet.

Die Auswertung der Einlaßventile erfolgt gravimetrisch. Dazu werden die Einlaßventile nach dem Ausbau an ihrer Unterseite sorgfältig mechanisch von Ablagerungen aus dem Verbrennungsraum befreit. Danach werden oberflächlich haftende, leicht lösliche Anteile auf den Ventilen durch Eintauchen in Cyclohexan entfernt und die Ventile durch Schwenken an der Luft getrocknet. Diese Behandlung wird insgesamt zweimal vorgenommen. Anschließend werden die Einlaßventile gewogen. Aus der Gewichtsdifferenz zwischen dem Ventilgewicht vor und nach dem Versuch ergibt sich die Menge an Ablagerungen pro Einlaßventil. Die Ergebnisse dieser Versuche sind in Tabelle 1 wiedergegeben.

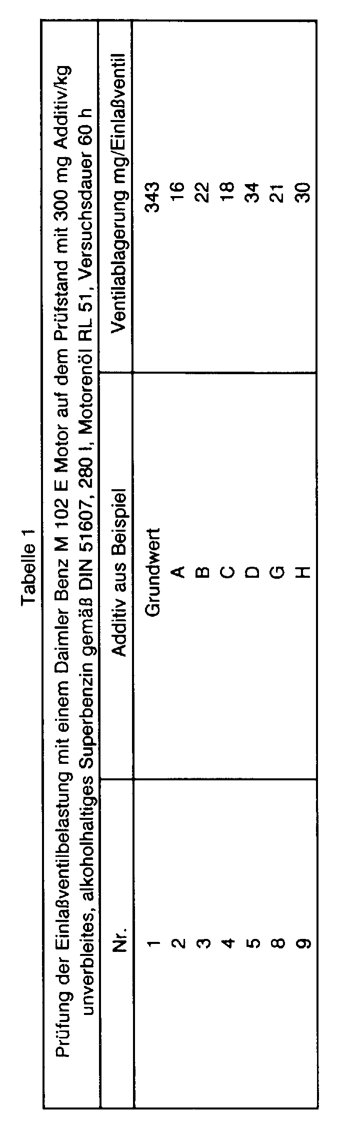

## Patentansprüche

1. Chlorfreie Kraftstoffe für Ottomotoren, enthaltend geringe Mengen eines Polyetheramins und/oder Polyetheraminderivats der allgemeinen Formel 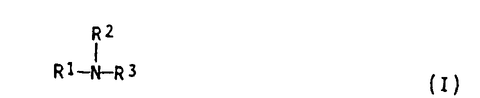 oder 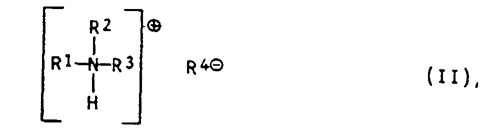 worin R¹ einen Alkylphenolpolyetherrest der allgemeinen Formel 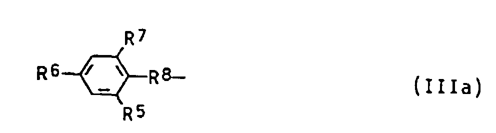 oder einen Alkylcyclohexylpolyetherrest der allgemeinen Formel 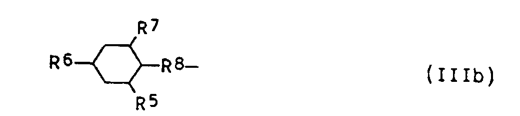 bedeutet, R² und R³ gleich oder verschieden sein können und für Wasserstoff, den Alkylphenolpolyetherrest (llla), den Alkylcyclohexylpolyetherrest (lllb), den Acylrest einer Carbonsäure mit 2 bis 24 Kohlenstoffatomen oder den Hydroxyalkylrest der allgemeinen Formel 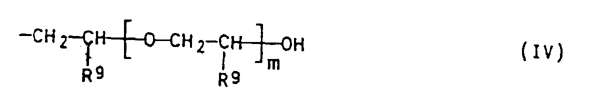 stehen oder R² für einen Alkylrest mit 1 bis 20 Kohlenstoffatomen steht und R³ für Wasserstoff, den Alkylphenolpolyetherrest (IIIa), den Alkylcyclohexylpolyetherrest (lllb), den Acylrest einer Carbonsäure mit 2 bis 24 Kohlenstoffatomen oder den Hydroxyalkylrest (IV) steht,
R⁴ den Carboxylatrest einer Carbonsäure mit 2 bis 24 Kohlenstoffatomen bedeutet, R⁵, R⁶ und R⁷ gleich oder verschieden sein können und Wasserstoff oder ganz oder teilweise Methyl- und/oder verzweigte Kohlenwasserstoffreste mit 3 bis 16 Kohlenstoffatomen bedeuten und wobei R⁵, R⁶ und R⁷ nicht gleichzeitig Wasserstoff bedeuten, R⁸ für eine Polyetherkette aus Propylenoxid und/oder Butylenoxid mit 2 bis 100 Alkenoxideinheiten in der Kette steht und R⁹ einen Kohlenwasserstoffrest mit 1 bis 6 Kohlenstoffatomen und m die Zahlen 0 bis 5 bedeuten, wobei das mittlere Molekulargewicht Mₙ der Polyetheramine oder Polyetheraminderivate (I) bzw. (II) 500 bis 8000 beträgt und die Polyetheramine oder die den Polyetheraminderivaten zugrunde liegenden Polyetheramine durch Aminierung von Alkylphenolpolyethern der allgemeinen Formel 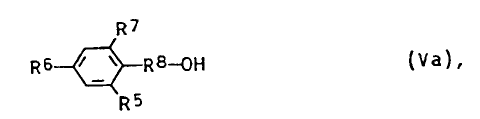 oder von Alkylcyclohexylpolyethern der allgemeinen Formel 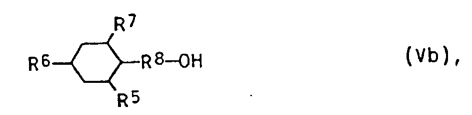 worin R⁵, R⁶, R⁷ und R⁸ die genannten Bedeutungen haben, mit Ammoniak oder primären Aminen hergestellt sind.

2. Kraftstoffe gemäß Anspruch 1, enthaltend ein Polyetheramin der Formel (I), in der R² und R³ Wasserstoff sind.

3. Kraftstoffe gemäß Anspruch 1, enthaltend ein Polyetheramin der Formel (I), in der R² Wasserstoff und R³ einen Acylrest bedeuten.

4. Kraftstoffe gemäß Anspruch 1, enthaltend ein Polyetheraminderivat der Formel (II), in der R⁴ der Carboxylatrest der iso-Nonansäure oder der Ethylhexansäure ist.

5. Kraftstoffe gemäß Anspruch 1 bis 4, enthaltend Polyetheramine und/oder Polyetheraminderivate, bei denen im Alkylphenolpolyetherrest der Formel (III) R⁵, R⁶ und R⁷ ganz oder teilweise Methyl- und/oder tert.-Butylreste darstellen.

6. Kraftstoffe gemäß Anspruch 1 bis 5, enthaltend Polyetheramine und/oder Polyetheraminderivate, bei denen im Alkylphenolpolyetherrest der Formel (III) R⁵ und R⁷ tert.-Butylreste und R⁶ einen Methylrest bedeuten.

7. Kraftstoffe gemäß Anspruch 1 bis 6, enthaltend Polyetheramine und/oder Polyetheraminderivate, die sich von Alkylphenolpolyethern der Formel (Va) ableiten, die durch Alkoxilierung der zugehörigen Alkylphenole mit Propylenoxid oder Butylenoxid oder mit einem Gemisch von Propylenoxid und Butylenoxid hergestellt sind.

8. Kraftstoffe für Ottomotoren gemäß Ansprüchen 1 bis 7, enthaltend 10 bis 2000 mg eines Polyetheramins und/oder Polyetheraminderivats der Formel (I) oder (II) pro kg Kraftstoff.

## Claims

1. A chlorine-free gasoline-engine fuel containing small amounts of a polyetheramine and/or a polyetheramine derivative of the formula 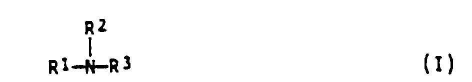 or 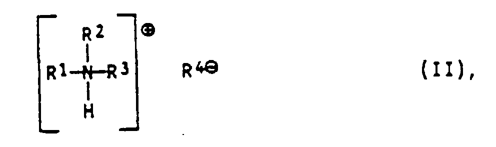 where R¹ is an alkylphenolpolyether radical of the formula 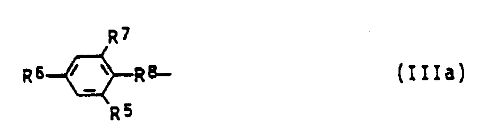 or an alkylcyclohexylpolyether radical of the formula 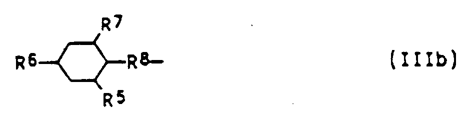 R² and R³ may be identical or different and are each hydrogen, an alkylphenolpolyether radical (IIIa), or an alkylcyclohexylpolyether radical (IIIb), an acyl radical of a carboxylic acid of 2 to 24 carbon atoms or a hydroxyalkyl radical of the formula 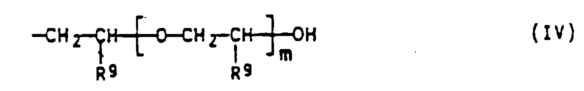 or R² is alkyl of 1 to 20 carbon atoms and R³ is hydrogen, an alkylphenolpolyether radical (IIIa), an alkylcyclohexylpolyether radical (IIIb), an acyl radical of a carboxylic acid of 2 to 24 carbon atoms or a hydroxyalkyl radical (IV), R⁴ is a carboxylate radical of a carboxylic acid of 2 to 24 carbon atoms, R⁵, R⁶ and R⁷ may be identical or different and are each hydrogen or some or all of them are methyl and/or branched hydrocarbon radicals of 3 to 16 carbon atoms, but R⁵, R⁶ and R⁷ are not simultaneously hydrogen, R⁸ is a polyether chain obtained from propylene oxide and/or butylene oxide having from 2 to 100 alkene oxide units in the chain, and R⁹ is a hydrocarbon radical of 1 to 6 carbon atoms and m is from 0 to 5, the mean molecular weight Mₙ of the polyetheramines or polyetheramine derivatives (I) or (II), respectively, being from 500 to 8,000 and the polyetheramines, or the polyetheramines on which the polyetheramine derivatives are based, being prepared by reductive amination of alkylphenolpolyethers of the formula 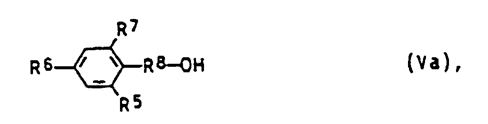 or of alkylcyclohexylpolyethers of the formula 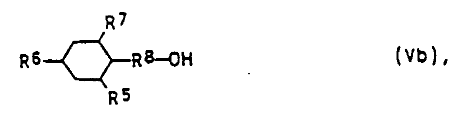 where R⁵, R⁶, R⁷ and R⁸ have the stated meanings, with ammonia or a primary amine.

2. A fuel as claimed in claim 1, containing a polyetheramine of the formula (I), where R² and R³ are each hydrogen.

3. A fuel as claimed in claim 1, containing a polyetheramine of the formula (I), where R² is hydrogen and R³ is acyl.

4. A fuel as claimed in claim 1, containing a polyetheramine derivative of the formula (II), where R⁴ is the carboxylate radical of isononanoic acid or of ethylhexanoic acid.

5. A fuel as claimed in any of claims 1 to 4, containing a polyetheramine and/or a polyetheramine derivative in which, in the alkylphenolpolyether radical of the formula (III), some or all of the radicals R⁵, R⁶ and R⁷ are methyl and/or tert-butyl radicals.

6. A fuel as claimed in any of claims 1 to 5, containing a polyetheramine and/or a polyetheramine derivative in which, in the alkylphenolpolyether radical of the formula (III), R⁵ and R⁷ are each tert-butyl and R⁶ is methyl.

7. A fuel as claimed in any of claims 1 to 6, containing a polyetheramine and/or a polyetheramine derivative which are derived from alkylphenolpolyethers of the formula (Va), which are prepared by oxyalkylation of the associated alkylphenols with propylene oxide or butylene oxide or with a mixture of propylene oxide and butylene oxide.

8. A gasoline-engine fuel as claimed in any of claims 1 to 7, containing from 10 to 2,000 mg of a polyetheramine and/or of a polyetheramine derivative of the formula (I) or (II) per kg of fuel.

## Revendications

1. Carburants exempts de chlore pour des moteurs à allumage par étincelles, contenant de faibles proportions d'une polyétheramine et/ou d'un dérivé de polyétheramine de la formule générale 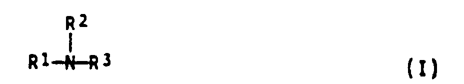 ou 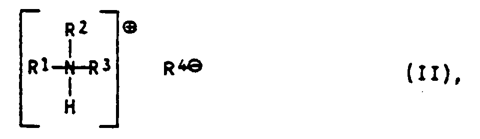 dans laquelle R¹ représente un reste d'alkylphénolpolyéther de la formule générale 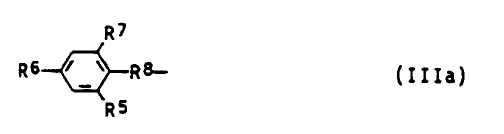 ou un reste d'alkylcyclohexylpolyéther de la formule générale 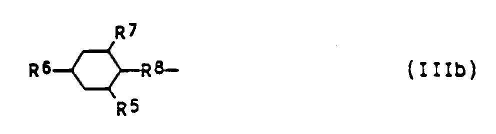 R² et R³ peuvent être identiques ou différents et représentent chacun un atome d'hydrogène, un radical alkylphénolpolyéther (IIIa), un radical alkylcyclohexylpolyéther (IIIb), le radical acyle d'un acide carboxylique comportant de 2 à 24 atomes de carbone, ou un radical hydroxyalkyle de la formule 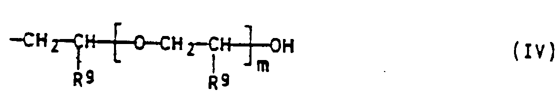 ou bien R² représente un radical alkyle comportant de 1 à 20 atomes de carbone et R³ représente un atome d'hydrogène, un radical alkylphénolpolyéther (IIIa), un radical alkylcyclohexylpolyéther (IIIb), le radical acyle d'un acide carboxylique comportant de 2 à 24 atomes de carbone, ou un radical hydroxyalkyle (IV),
R⁴ représente le reste carboxylate d'un acide carboxylique comportant de 2 à 24 atomes de carbone, R⁵, R⁶ et R⁷ peuvent être identiques ou différents et représentent chacun un atome d'hydrogène ou totalement ou en partie des radicaux méthyle et/ou hydrocarbonés ramifiés comportant de 3 à 16 atomes de carbone et où R⁵, R⁶ et R⁷ ne représentent pas ensemble de l'hydrogène, R⁸ représente une chaîne polyéther constituée d'oxyde de propylène et/ou d'oxyde de butylène comportant de 2 à 100 unités oxyde d'alcène dans la chaîne et R⁹ représente un reste hydrocarboné comportant de 1 à 6 atomes de carbone et m représente les nombres 0 à 5, où le poids moléculaire moyen Mₙ des polyétheramines ou des dérivés de polyétheramine (I) ou (II) varie de 500 à 8000 et les polyétheramines ou les polyétheramines qui sont à la base des dérivés de polyétheramine sont préparées par l'amination d'alkylphénolpolyéthers de la formule générale 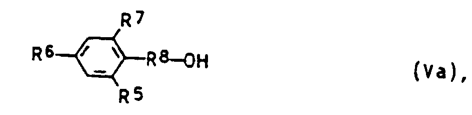 ou d'alkylcyclohexylpolyéthers de la formule générale 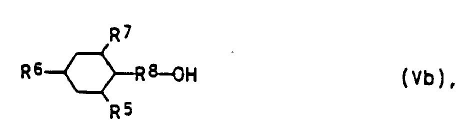 dans lesquelles R⁵, R⁶, R⁷ et R⁸ possèdent les significations qui leur ont été précédemment attribuées, à l'aide d'ammoniac ou d'amines primaires.

2. Carburants suivant la revendication 1, caractérisés en ce qu'ils contiennent une polyétheramine de la formule (I) dans laquelle R² et R³ représentent des atomes d'hydrogène.

3. Carburants suivant la revendication 1, caractérisés en ce qu'ils contiennent une polyétheramine de la formule (I) dans laquelle R² représente un atome d'hydrogène et R³ représente un radical acyle.

4. Carburants suivant la revendication 1, caractérisés en ce qu'ils contiennent un dérivé de polyétheramine de la formule (II) dans laquelle R⁴ représente le reste carboxylate de l'acide isononanoïque ou de l'acide éthylhexanoïque.

5. Carburants suivant l'une quelconque des revendications 1 à 4, caractérisés en ce qu'ils contiennent des polyétheramines et/ou des dérivés de polyétheramine chez lesquels dans le reste alkylphénopolyéther de la formule (III), R⁵, R⁶ et R⁷ représentent totalement ou partiellement des radicaux méthyle et/ou tert-butyle.

6. Carburants suivant l'une quelconque des revendications 1 à 5, caractérisés en ce qu'ils contiennent des polyétheramines et/ou des dérivés de polyétheramine chez lesquels dans le reste alkylphénolpolyéther de la formule (III), R⁵ et R⁷ représentent des radicaux tertbutyle et R⁶ représente le radical méthyle.

7. Carburants suivant l'une quelconque des revendications 1 à 6, caractérisés en ce qu'ils contiennent des polyétheramines et/ou des dérivés de polyétheramine, qui dérivent d'alkylphénolpolyéthers de la formule (Va), qui ont été préparés par l'alcoxylation des alkylphénols apparentés avec de l'oxyde de propylène ou de l'oxyde de butylène ou avec un mélange d'oxyde de propylène et d' oxyde de butylène.

8. Carburants pour moteurs à allumage par étincelles suivant l'une quelconque des revendications 1 à 7, caractérisés en ce qu'ils contiennent de 10 à 2000 mg d'une polyétheramine et/ou d'un dérivé de polyétheramine de la formule (I) ou (II) par kg de carburant.
